# EUROPÄISCHE PATENTANMELDUNG

(11) **EP 0 534 246 A2**
(43) Veröffentlichungstag der Anmeldung: **31.03.1993**
(21) Anmeldenummer: 92115551.1
(22) Anmeldetag: 11.09.1992
(51) Int. Cl.: C07C 233/80, A61K 31/165, C07C 255/57, C07C 311/08, C07C 217/58, C07C 311/21, C07C 255/54, C07D 213/38, C07D 295/096, C07D 295/135, A61K 31/445

(54) **Benzylaminederivate und diese enthaltende Arzneimittel mit Antiarhythmischer Wirkung**

(30) Priorität: 26.09.1991 DE 4132013
(71) Anmelder: BASF Aktiengesellschaft, D-67063 Ludwigshafen (DE)
(72) Erfinder: Lubisch, Wilfried, Dr., W-6800 Mannheim 1 (DE); Schult, Sabine, Dr., W-6900 Heidelberg (DE); Binder, Rudolf, Dr., W-6520 Worms (DE); Raschack, Manfred, Dr., W-6714 Weisenheim am Sand (DE); Reinhardt, Roland, Dr., W-6750 Kaiserslautern (DE); Seemann, Dietmar, Dr., W-6907 Nussloch (DE)

(57) **Zusammenfassung**

Phenylbenzylamine der Formel I 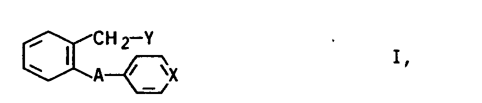
worin bedeuten:
A 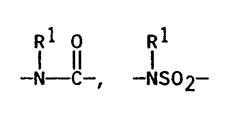
   und -O(CH₂)m- (mit Bindung von N bzw. O an den 1,2-Phenylenring)
   R¹ H und G₁-C₄-Alkyl,
   X N und C-R²,
   R² N0₂, CN, NHS0₂CH₃, CF₃, OCF₃ und 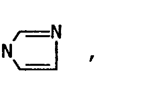
   Y NR¹R³ und N (CH₂)ₙ,
   n 4,5 und 6,
   R³ H, G₁-C₄-Alkyl, 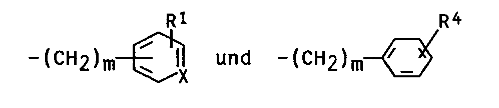
   m 1, 2 und 3 und
   R⁴ H, OCH₃ und C₁-C₄-Alkyl,

   sowie deren physiologisch verträglichen Salze und daraus hergestellte Arzneimittel zur systemischen und topischen Anwendung.

## Beschreibung

Die Erfindung betrifft neue substituierte Benzylaminderivate und diese als Wirkstoff enthaltende Arzneimittel.

Die Erfindung bezieht sich auf Benzylaminderivate der Formel I 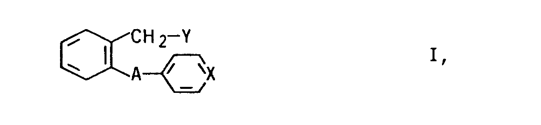
worin bedeuten:
A 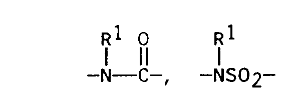
   und -O(CH₂)m- (mit Bindung von N bzw. O an den 1,2-Phenylenring) R¹ H und G₁-C₄-Alkyl,
   X N und C-R²,
   R² N0₂, CN, NHS0₂CH₃, CF₃, OCF₃ und 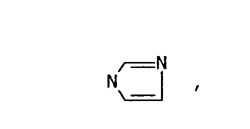
   Y NR¹R³ und N (CH₂)ₙ ,
   n 4, 5 und 6,
   R³ H, G₁-C₄-Alkyl, 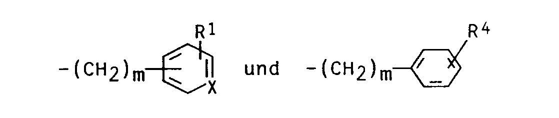
   m 1, 2 und 3 und
   R⁴ H, OCH₃ und C₁-C₄-Alkyl,

sowie deren physiologisch verträglichen Salze.

Die erfindungsgemäßen Verbindungen können auf verschiedenen konventionellen Wegen dargestellt werden.

Entsprechend dem Schema 1 wird das Amin HY mit 2-Nitrobenzylchlorid II unter üblichen Bedingungen in polaren Lösungsmitteln, vorzugsweise Alkoholen, in Gegenwart von Basen wie Kaliumkarbonat und bei Temperaturen von ca. 25-100°C alkyliert, wobei das Benzylamin III anfällt. III kann ebenso durch reduzierende Aminierung des 2-Nitrobenzaldehyds VI mit dem Amin HY in polaren Lösungsmitteln, vorzugsweise Alkoholen, in Gegenwart von Natriumcyanoborhydrid erhalten werden. III wird in konventioneller Weise zum Anilin IV reduziert, wobei als Reduktionsmittel Wasserstoff in Gegenwart eines Katalysators wie Pd/Kohle, Pt/Kohle oder Raney-Nickel oder Reagenzien, die z.B. im Houben-Weyl, Methoden der organischen Chemie, Bd. 11/1, Kap. IV aufgelistet sind, z.B. Sn/HCI, Fe/HCI und Na₂S₂0₄, eingesetzt werden. IV wird mit Säurechloriden 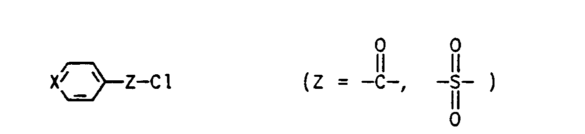
unter üblichen Bedingungen (gelöst in organischen Lösungsmitteln wie Methylenchlorid oder Tetrahydrofuran in Gegenwart einer Base wie Natronlauge, wäßriger Kaliumkarbonatlösung oder Triethylamin bei Temperaturen von -10 bis 25 °C) umgesetzt. Man erhält die erfindungsgemäßen Amide bzw. Sulfonamide V. Das Anilin IV kann ebenso aus dem Benzaldehyd VI erhalten werden. VI wird dazu mit einem Amin R³NH₂ in Lösung, bevorzugt in Alkoholen, bei 25-100°C umgesetzt. Das so erhaltene Imin VII wird in üblicher Weise, bevorzugt mit Natriumborhydrid in Alkohol bei Raumtemperatur, zum sekundären Amin VIII reduziert. Die Alkylierung von VIII zu III erfolgt entweder mit einem Halogenid R¹-Hal (Hal=CI, Br und J) in Lösungsmitteln wie Alkoholen bei Temperaturen von 25-150°C oder als reduktive Aminierung mit einem Aldehyd R¹-CHO in Gegenwart von Reduktionsmitteln wie Natriumcyanborhydrid in Lösungsmitteln wie Alkoholen bei Temperaturen von 0-60°C.

III wird dann wie oben beschrieben zu IV reduziert.

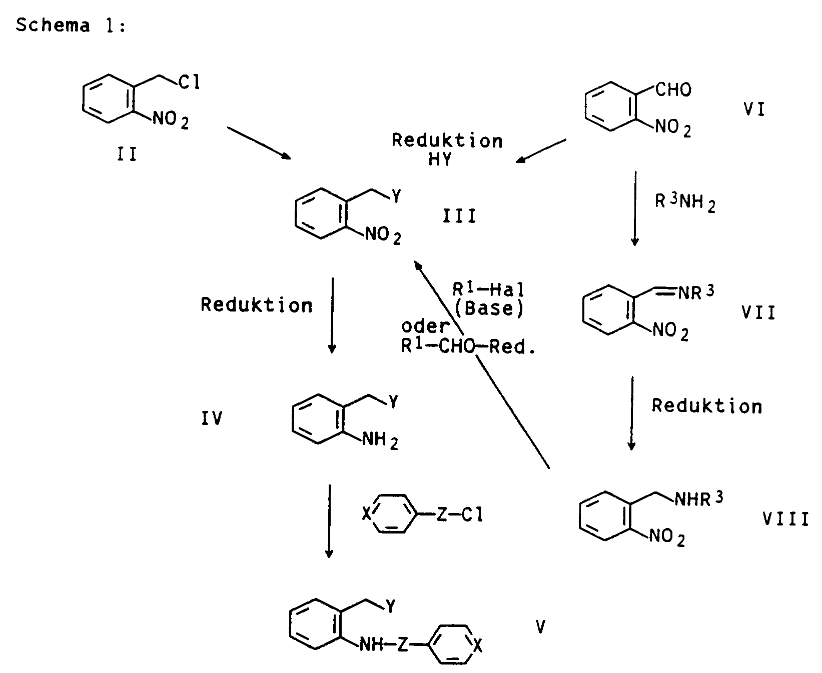

Die Synthese der erfindungsgemäßen Verbindungen XII ist im Schema 2 dargestellt. Salicylaldehyd wird mit einem Aralkyl 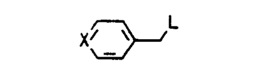
alkyliert, wobei L eine Abgangsgruppe wie CI oder O-Tosylat ist. Die Reaktion wird in Lösung, bevorzugt in Alkoholen oder Dimethylformamid, in Gegenwart von Basen wie Kaliumkarbonat durchgeführt. Der Aldehyd IX kann durch reduktive Aminierung, die in Lösungsmitteln wie Alkoholen in Gegenwart von Reduktionsmitteln wie Natriumcyanborhydrid ausgeführt wird, mit HY zum Endprodukt XII umgesetzt werden.

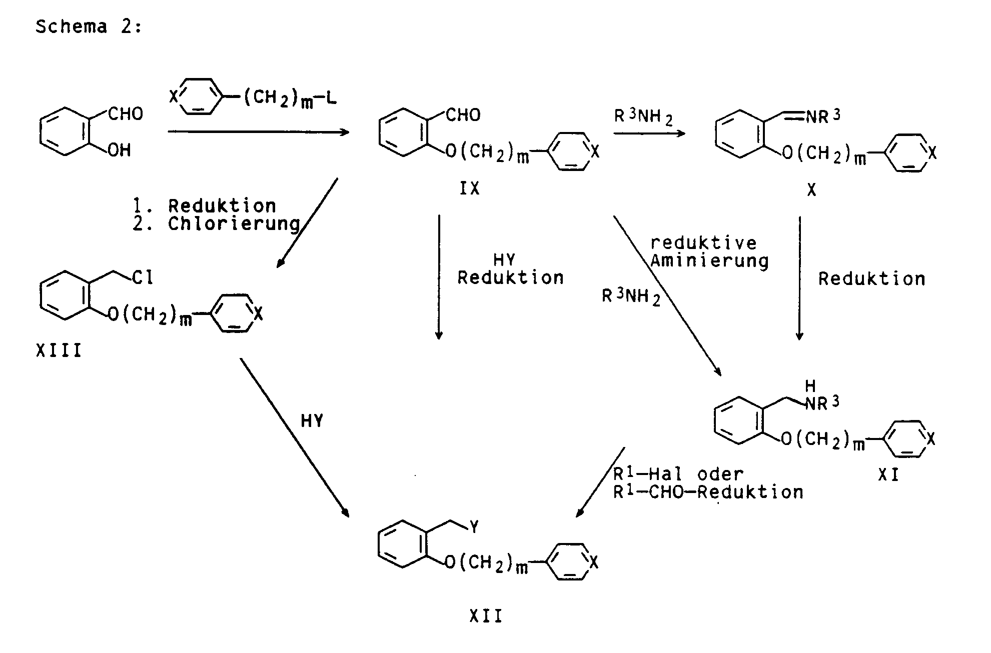

Bei der Reaktion des Aldehyds IX mit dem Amin R³NH₂, die in Alkoholen bei Temperaturen von 25 bis 100°C ausgeführt wird, erhält man das Imin X, das durch Reduktion mit Natriumborhydrid in Lösungsmitteln wie Alkohole das Amin XI ergibt. Die überführung von XI in das Endprodukt XII erfolgt entweder durch Alkylierung mit einem Halogenid R^{l-}Hal (Hal=Chlor, Brom und Jod) in polaren Lösungsmitteln wie Alkoholen oder Dimethylformamid bei Temperaturen von 25 bis 150°C, oder durch reduktive Aminierung mit einem Aldehyd R¹CHO in Lösungsmitteln wie Alkoholen in Gegenwart von Reduktionsmitteln wie Natriumcyanborhydrid.

Das Benzaldehyd-Derivat IX kann durch Reduktion, bevorzugt mit Natriumborhydrid in Alkoholen, und anschließende Chlorierung mit oder ohne Lösungsmittel mit Chlorierungsmitteln wie Thionylchlorid in das Benzylchlorid XIII überführt werden. Die nachfolgende Alkylierung des Amins HY mit dem Chlorid XIII zur erfindungsgemäßen Verbindung XII erfolgt in Alkoholen oder Dimethylformamid in Gegenwart von Basen wie Kaliumkarbonat.

Gegebenenfalls werden die so erhaltenen Benzylaminderivate in das Säureadditionssalz einer physiologisch verträglichen Säure übergeführt. Eine Zusammenstellung üblicher physiologisch verträg-licher Säuren kann aus Fortschritte der Arzneimittelforschung, 1966, Birkhäuser Verlag, Bd. 10, S. 224 bis 285, Deutschland, Schweiz, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol, wie Methanol, Ethanol oder Propanol, oder einem niederen Keton, wie Aceton, Methylethylketon oder Methylisobutylketon, oder einem Ether wie Diethylether, Tetrahydrofuran oder Dioxan, erhalten. Zur besseren Kristallabscheidung können Mischungen der genannten Lösungsmittel verwendet werden. Darüber hinaus können pharmazeutisch vertretbare waßrige Lösungen von Säure-Additionsverbindungen der Benzylaminderivate der Formel 1 durch Auflösen der freien Basen in einer wäßrigen Säurelösung hergestellt werden.

Die erfindungsgemäßen Benzylaminderivate stellen Antiarrhythmika der Klasse III dar. Zudem zeigen sie Affinität zum Sigma-Rezeptor und daher antipsychotische, anxiolytische, antikonvulsive und neuroprotektive Wirkung. Weiterhin wurde gefunden, daß die Verbindungen den ATP-sensitiven K-Kanal blockieren.

Die Erfindung betrifft daher weiter therapeutische Mittel zur topischen und vor allem systemischen Anwendung, die eine Verbindung der Formel 1 neben üblichen Trägerstoffen und/oder sonstigen galenischen Hilfsmitteln als Wirkstoff enthalten, und die Verwendung einer Verbindung der Formel 1 zur Herstellung eines Arzneimittels.

Die Herstellung der therapeutischen Mittel oder Zubereitungen erfolgt mit den üblichen flüssigen oder festen Trägerstoffen oder Verdünnungsmitteln und den üblicherweise verwendeten pharmazeutisch-technischen Hilfsstoffen, entsprechend der gewünschten Applikationsart und mit einer zur Anwendung geeigneten Dosierung, in üblicher Weise, beispielsweise durch Vermischen des Wirkstoffes mit den an sich in solchen Präparaten üblichen festen oder flüssigen Träger- und Hilfsstoffen.

Die Mittel können peroral, parenteral oder topisch verabreicht werden. Derartige Zubereitungen sind beispielsweise Tabletten, Filmtabletten, Dragees, Kapseln, Pillen, Pulver, Lösungen oder Suspensionen, Infusions-oder Injektionslösungen sowie Pasten, Salben, Gele, Cremes, Lotionen, Puder, Lösungen oder Emulsionen und Sprays.

Die therapeutischen Mittel können die erfindungsgemäß zu verwendenden Verbindungen bei lokaler Anwendung in 0,001 bis 1 %iger Konzentration, bevorzugt in 0,001 bis 0,1 %iger Konzentration, und bei systemischer Anwendung vorzugsweise in einer Einzeldosis von 0,1 bis 50 mg pro kg Körpergewicht enthalten und täglich in einer oder mehreren Dosierungen je nach Art und Schwere der Erkrankungen verabreicht werden.

üblicherweise verwendete pharmazeutisch-technische Hilfsstoffe sind beispielsweise für die lokale Anwendung Alkohole, wie Ethanol, Isopropanol, oxethyliertes Ricinusöl oder oxethyliertes hydriertes Ricinusöl, Polyacrylsäure, Glycerinmonostearat, Paraffinöl, Vaseline, Wollfett, Polyethylenglykol, Polypropylenglykol, Salze der Stearinsäure sowie ethoxylierter Fettalkohol, für die systemische Anwendung Milchzucker, Propylenglykol und Ethanol, Stärke, Talk, Polyvinylpyrrolidon. Den Präparaten können Antioxidationsmittel, beispielsweise Tocopherol sowie butyliertes Hydroxyanisol oder butoxyliertes Hydroxytoluol, oder geschmacksverbessernde Zusätze, Stabilisierungsmittel, Emulgiermittel, Bleichmittel usw. zugesetzt werden. Voraussetzung ist, daß alle bei der Herstellung pharmazeutischer Zubereitungen verwendeten Stoffe toxikologisch unbedenklich und mit den verwendeten Wirkstoffen verträglich sind.

Die therapeutischen Wirkungen können anhand folgender Modelle ermittelt werden:

### Antiarrhythmische Wirkung

Die Wirkung der Benzylaminderivate als Repolarisationshemmstoffe kann man durch EKG-Messungen belegen. Dabei unterteilt man die Herzperiode zeitlich in Systole (Zusammenziehen des Herzens), auch QT-Dauer genannt, und Diastole (Erschlaffung des Herzens mit Blutfüllung der Herzkammern). Repolarisationshemmstoffe bewirken nun eine Verlängerung der QT-Dauer, ohne die Vorhofkammer-überleitungszeit (PQ-Dauer) und die Anspannungszeit (QRS-Dauer, Beginn der Systole bis zum Öffnen der Semilunarklappen) wesentlich zu verändern.

Die Wirksamkeit der erfindungsgemäßen Verbindungen als Repolarisationshemmstoffe läßt sich im Tierversuch durch EKG-Messungen beispielsweise am Meerschweinchenherzen untersuchen (s. Basic Res. Cardiol. 82 (1987) 437; J. Pharmacol. Methods 21 (1989) 195). Zum Vergleich der Wirksamkeit mehrerer Substanzen dient dabei zum Beispiel die Dosis eines Wirkstoffes, bei der eine 20 %ige Zunahme des QT-Ausgangswertes eintritt (ED_{20 %}). Hierzu trägt man die logarithmierten Dosiswerte der jeweiligen Substanzen gegen die experimentell gefundenen relativen Änderungen der QT-Dauer auf, und ermittelt mittels linearer Regression die Funktionsgleichung einer Geraden, aus der man dann den ED_{20 %}-Wert berechnen kann.

Als Versuchstiere dienten männliche Duncin-Hartley-Meerschweinchen mit einem Körpergewicht von 300 bis 350 g. 30 min nach Applikation von 1250 1.E. Heparin/kg Körpergewicht in den Bauchraum wurden die Tiere durch Genickschlag getötet. Nach dem Durchtrennen der Kopfschlagadern zum Entbluten wurde der Bruststamm geöffnet, das Herz herausgetrennt und an eine Perfusionsapparatur angeschlossen. Die Perfusion erfolgte nach Langendorff mit an Sauerstoff angereicherter, auf 37 ° C erwärmter Krebs-Henseleit-Lösung (NaCI 6896 mg/I; KCI 350 mg/I; MgS0₄ 285 mg/I; CaC1₂ 370 mg/I; KH₂ P0₄ 161 mg/I: NaHC0₃ 2090 mg/I; Glukose 2000 mg/I). Dabei wurde das Perfusionsvolumen pro Zeiteinheit bei einem Gesamtvolumen von 100 ml auf 4 bis 6 ml/min und der Perfusionsdruck auf 60 bis 70 mm Hg eingestellt. Nach einer Äquilibrierzeit von 30 min wurde zirkulierend perfundiert.

Die EKG-Messungen wurden über zwei Silberelektroden, die auf der Herzoberfläche im oberen Bereich der linken Koronararterie und auf der Rückseite des Herzens auf Höhe der Ventilebene angebracht waren, aufgenommen. Gemessen wurden die PQ-, QT- und QRS-Zeiten sowie die Herzfrequenz.

Die Substanzgabe erfolgte kumulativ im Abstand von 15 min ins Perfusat.

### Bindung an Sigma-Rezeptor

Sigma-Rezeptoren wurden vor wenigen Jahren entdeckt. Sie eröffnen neue Möglichkeiten zur Therapie verschiedener Krankheiten (vgl. C.D. Ferris et al., J. Neurochemistry, 57 (3), 729-737 (1991)). Zum Beispiel könnten Sigma-Liganden als Antipsychotika, Antikonvulsiva, Anxiolytika und Neuroprotektiva eingesetzt werden. Eine Reihe von beschriebenen Verbindungen wie Haloperidol und BMY 14802 zeigen Affinität zum Sigma-Rezeptor (B.L. Largent et al., J. Pharmacol. Exp. Ther. 2, 739-748 (1986)), wobei jedoch diese Vertreter auch Affinitäten zu anderen Rezeptoren wie den dopaminergen und serotoninergen Rezeptoren aufweisen. Es werden daher neue Vertreter mit spezifischer Wirkung auf die Sigma-Rezeptoren gesucht. Die Affinitäten der erfindungsgemäßen Verbindungen zu den Sigma-Rezeptoren wurden anhand der Verdrängung von ³H-Ditolylguanidin (Bindungsassay) gemessen.

Als Maß für die Potenz der Verbindungen wurden die Affinitätskonstanten K angegeben, die durch iterative Anpassungsprogramme aus den Verdrängungsexperimenten ermittelt wurden.

Der verwendete Bindungsassay (Bindung von [³H]-Ditolylguanidin) erfaßt sogenannte "Haloperidolsensitive-Sigma-Rezeptoren", die eine hohe Affinität zum Haloperidol, aber nur geringe Affinität zum Phencyclidin und zu Opoiden aufweisen.

### Methoden:

### a) Membranpreparation

Rattengroßhirne wurden in dem 10fachen Volumen Homogenisierungspuffer (50 mmol Tris-(hydroxymethyl)-amino-methan, 0,1 mmol Ethylendiamintetraacetat, pH = 7,7) mit einem Polytron-Homogenisator homogenisiert (20 sec.). Nach 15 Minuten Zentrifugation bei 40000 Umdrehungen/min wurde das erhaltene Pellet resuspendiert und die Suspension erneut 15 Minuten lang mit 40000 Umdrehungen/min zentrifugiert. Das hierbei erhaltene Pellet wurde im 5fachen Volumen Homogenisierungspuffer resuspendiert und bis zur weiteren Verwendung in flüssigem Stickstoff eingefroren.

### ß) Sigma-Bindungstest

Testsubstanz und Membranen (0,3 mg Protein) wurden in 0,3 ml Inkubationspuffer (5 mmol Tris-(hydroxymethyl)-amino-methan, 0,1 mmol Ethylendiamintetraacetat, pH = 7,7) 45 Minuten bei 37°C inkubiert. Nach Zugabe von 100 000 dpm [³H]-Ditolylguanidin (54,5 Ci/mmol) wurde noch 1 Stunde inkubiert. Die Membranen wurden über GF/B-Filter (dunn-Labortechnik, Asbach) filtriert und mit einem 37°C warmen Waschpuffer (5 mmol Tris(hydroxymethyl)-amino-methan, 0,1 mmol Ethylendiamintetraacetat, pH=7,4) gewaschen. Die verbleibende Radioaktivität auf den Filtern wurde durch Flüssig-Scintillationszählung gemessen. Die Bindungsdaten wurden durch iterative Anpassungsprogramme analysiert.

### ATP-abhängiger K -Strom

An isolierten ventrikulären Myozyten des Meerschweinchens wurde der ATP-abhängige K⁺-Strom (A. Noma, Nature 305, 147-148 (1983)) mit der Patch-Clamp-Technik in der whole-cell-Konfiguration (O. P. Hamill et al., Pflügers Arch. 391, 85-100 (1981)) gemessen. Mit einer Spannungsrampe von -100 mV bis 60 mV wurde die I/V-Kurve des gesamten K -Stromes der Ventrikelzellen aufgenommen. Der ATP-abhängige K⁺-Strom wurde entweder mit Dinitrophenol (W.J. Lederer et al., J. Physiol. (London) 413, 329-349 (1989)) oder mit Cromakalim (D. Escande et al., Biochem. Biophys. Res. Comm. 154, 620-625 (1988)) aktiviert. Ein selektiver Blocker für den ATP-abhängigen K⁺-Strom in Ventrikelzellen und ß-Zellen ist der Sulfonylharnstoff Glibenclamid (S.J.H. Ashcroft et al., Cellular Signalling 2, 197-214 (1990)). Für Glibenclamid konnte in vitro und in vivo antiarrhytmische bzw. antifibrillatorische Wirkung nachgewiesen werden (S.S. Bekheit et al., Am. Heart J. 119 (5), 1025-33 (1990); C.D. Wolleben et al., J. Mol. Cell. Cardiol. 21, 783-88 (1989)). Außerdem reduziert Glibenclamid die ischämische extracelluläre K⁺-Akkumulation (S.S. Bekheit et al., loc. cit.). Diese Effekte zeigen ein antiarrhytmisches/antiischämisches therapeutisches Potential für K⁺_{ATP}-Kanal-Blocker. Da Glibenclamid eine wesentlich stärkere Wirkung an ß-Zellen ausübt (S.J.H. Ashcroft et al., loc. cit.), eignet es sich nicht für kardiale Anwendungen. Neuartige Blocker dieses lonenkanals sind daher wünschenswert. Einen neuartigen, nicht Sulfonylharnstoff-artigen Blocker dieses lonenkanals stellt das 5-Hydroxydekanoat dar, das aber erst bei einer Konzentration von ca. 100 um Wirkung zeigt (J. Mol. Cell. Cardiol. 21, Suppl. 2, S. 9, 1989).

### Vorprodukt-Präparate

### Präparation 1:

20,0 g (0,12 mol) 2-Nitrobenzylchlorid, 14,2 g (0,12 mol) N,N-Benzylmethylamin und 34,2 g Kaliumkarbonat wurden in 300 ml Ethanol 5 h bei 60°C gerührt. Man filtrierte anschließend den Niederschlag ab und engte das Filtrat i.Vak. ein. Der Rückstand wurde in Toluol gelöst und mit etherischer Chlorwasserstoff-Lösung versetzt. Das ausgefallene Öl wurde aus wenig Aceton kristallisiert. Man erhielt 26,2 g N,N,N-Benzyl-methyl-(2-nitrobenzyl)aminhydrochlorid.

Schmp. 184-186 °C.

### Präparation 2:

26 g (0,10 mol) der Base des Produkts aus der Präparation 1 wurden in 250 ml Methanol in Gegenwart von 1 g Platin/Kohle (5 %) in üblicher Weise hydriert. Man erhielt 20,6 g N,N,N-(2-Aminobenzyl)-benzylmethylamin als Öl. ¹H-NMR (CDCI₃: 0 = 2,1 (s, 3H), 3,4 (s, 2H), 3,5 (s, 2H), 4,7 (breit, 2H, NH) und 6,6-7,4 (9H) ppm.

### Präparation 3:

15,8 g (0,12 mol) N,N-Methyl-(2-phenylethyl)amin, 20,0 g (0,12 mol) 2-Nitrobenzylchlorid und 34 g Kaliumkarbonat wurden analog zur Präparation 1 zugesetzt. Man erhielt 29 g N,N,N-Methyl-(2-nitrobenzyl)-(2-phenylethyl)amin, das als Rohprodukt direkt weiter umgesetzt wurde. ¹H-NMR (CDCI₃: 0 = 2,2 (s, 3H), 2,6-2,7 (m, 2H), 2,7-2,9 (m, 2H) und 7,0-7,8 (9H) ppm.

### Präparation 4:

26 g (96,2 mmol) des Rohproduktes aus der Präparation 3 wurden analog Präparation 2 an Pt/Kohle (5 %) in Methanol hydriert. Man erhielt 22,4 g des öligen N,N,N-(2-Aminobenzyl)-methyl-(2-phenylethyl)amins. ¹HNMR (D₆-DMSO): 0 = 2,1 (s, 3H), 2,5-2,7 (m, 2H), 2,7-2,9 (m, 2H), 3,4 (s, 2H), 5,0 (2H, NH), 6.5 (t, 1H), 6,6 (d, 1H), 6,9-7,0 (2H) und 7,1-7,3 (5H) ppm.

### Präparation 5:

20,6 g (0,08 mol) 2-(4-Nitrobenzyloxy)benzaldehyd wurden in einem Gemisch aus 200 ml Isopropanol und 50 ml Methanol gelöst und anschließend nacheinander mit 200 ml Wasser und 6,0 g (0,16 mol) Natriumborhydrid versetzt. Nach 16stündigem Rühren bei Raumtemperatur saugte man die ausgefallenen Kristalle ab. Man erhielt 16,4 g 2-(4-Nitrobenzyloxy)-benzylalkohol. Schmp. 123°C.

### Präparation 6:

34,0 g (0,13 mol) des Produktes aus Präparation 5 wurden in 350 ml Methylenchlorid suspendiert und mit 15 ml Thionylchlorid versetzt. Man kochte alles 2 h am Rückfluß und engte anschließend das Reaktionsgemisch i. Vak. ein. Man erhielt 36,9 g 2-(4-Nitrobenzyloxy)benzylchlorid. Schmp. 111 °C.

### Präparation 7:

2,5 g (10 mmol) 2-(4-Cyanobenzyloxy)benzaldehyd und 1,2 g (10 mmol) 4-(2-Aminoethyl)pyridin wurden 8 h in 50 ml Ethanol unter Rückfluß gekocht. Man engte anschließend alles i. Vak. ein und kristallisierte den Rückstand aus Cyclohexan/wenig Toluol um. Man erhielt 2,4 g 2-(2-(4-Cyanobenzyloxy)-phenyl)N-(2-(4-pyridyl)-ethyl)-methylenimin.

¹ H-NMR(CDCI₃) 0 = 3.0(t,2H); 3.9(t,2H) ; 5.1 (s,2H); 6.9(d,1 H); 7.0(t,1 H); 7.1 (d,2H); 7.4(dt,1 H); 7.5(d,2H); 7.7(d,2H); 8.0(dd,1 H); 8.5(d,2H) und 8.6(s,1 H) ppm.

### Beispiel 1

1,9 g (8,4 mmol) des Produktes aus der Präparation 2 wurden in 50 ml Methylenchlorid gelöst. Man gab 50 ml 2 M Natronlauge und anschließend bei 0-5°C tropfenweise 1,9 g (10,2 mmol) 4-Nitrobenzoylchlorid, gelöst in Methylenchlorid, zu, ließ 1 h rühren und trennte die organische Phase ab, die danach noch mit H₂0 gewaschen, getrocknet und mit etherischer Chlorwasserstoff-Lösung versetzt wurde. Der beim Eindampfen erhaltene Rückstand wurde mit wenig Aceton aufgekocht. Man erhielt 2,9 g N-(2-(N,N-Benzylmethylaminomethyl)phenyl)-4-nitrobenzamidhydro-chlorid; Schmp. 230 C.

### Beispiel 2

3,0 g (12,5 mmol) des Produktes aus der Präparation 4 wurden mit 2,4 g (14,5 mmol) 4-Cyanbenzoylchlorid analog dem Beispiel 1 umgesetzt. Man erhielt 2,4 g 4-Cyano-N-(2-((N,N-methyl-(2-phenylethyl)-amino)methyl)phenyl)-benzamid. Schmp. 128°C (Isopropanol).

### Beispiel 3

3,0 g (12,5 mmol) des Produktes aus der Präparation 4 und 2,5 g (25 mmol) Triethylamin wurden in 100 ml wasserfreiem Tetrahydrofuran gelöst. Bei 0-5 ° C tropfte man 2,75 g (12 mmol) 4-Methansulfonylaminobenzylchlorid, gelöst in Tetrahydrofuran, zu. Man rührte 2-3 h und engte das Reaktionsgemisch anschließend i. Vak. ein. Der Rückstand wurde aus wenig Isopropanol umkristallisiert. Man erhielt 3,8 g 4-Methansulfonylamino-N-(2-((N,N-methyl-(2-phenylethyl)amino)-methyl)phenyl)benzamid. Schmp. 153 ° C.

### Beispiel 4

3,0 g (10,8 mmol) des Produktes aus der Präparation 6, 1,3 g (10,8 mmol) N,N-Benzylmethylamin und 2 g Kaliumkarbonat wurden analog der Präparation 1 umgesetzt. Das gewonnene Produkt wurde als Fumarat aus Ethanol kristallisiert. Man erhielt 4,1 g N,N,N-Benzylmethyl-(2-(4-nitrobenzyloxy)benzyl)-amin- fumarat. Schmp. 157°C.

### Beispiel 5

4,0 g (14,4 mmol) des Produktes aus der Präparation 6, 2,0 g (14,8 mmol) N,N-Methyl-(2-phenylethyl)-amin und 2 g Kaliumkarbonat wurden analog der Präparation 1 umgesetzt. Man erhielt 3,6 g N,N,N-Methyl-(2-(4-nitrobenzyloxy)benzyl)-(2-phenylethyl)aminhydrochlorid. Schmp. 195 C.

### Beispiel 6

3,0 g (12,7 mmol) 2-(4-Cyanobenzyloxy)benzaldehyd, 1,7 g (12,7 mmol) und 0,76 g (12,7 mmol) Eisessig wurden in iso-Propanol gelöst und bei Raumtemperatur gerührt. Man gab portionsweise 0,8 g (12,7 mmol) Natriumcyanoborhydrid zu und rührte weitere 16 h. Anschließend wurde das Reaktionsgemisch im Vakuum eingeengt, der Rückstand zwischen 2 M Natronlauge und Ether verteilt und die organische Phase getrocknet. Die Etherphase wurde mit etherischer Chlorwasserstoff-Lösung versetzt und das ausgefallene Produkt aus iso-Propanol umkristallisiert. Man erhielt 2,4 g N,N,N-(2-(4-Cyanobenzyloxy)benzyl)-methyl-(2-phenylethyl)-amin-hydrochlorid. Schmp. 185°C.

### Beispiel 7

Zu 2,4 g (7,0 mmol) des Produktes aus der Präparation 7, das in 100 ml Ethanol gelöst wurde, gab man bei Raumtemperatur portionsweise 0,27 g (7,1 mmol) Natriumborhydrid. Man rührte 16 h und engte anschließend das Reaktionsgemisch im Vakuum ein. Der Rückstand wurde zwischen Methylenchlorid und Wasser verteilt, die organische Phase getrocknet und im Vakuum eingeengt. Man erhielt 2,5 g N,N-(2(4-Cyanobenzyloxy)-benzyl-(2-(4-pyridyl)ethyl)amin.

¹H-NMR (D₂0) 0 = 3.2-3.8(4H); 4.4(s,2H); 5.3(s,2H); 7.1(2H); 7.3-7.8(6H) und 8.8(2H) ppm.

### Beispiel 8

2,4 g (7 mmol) des Produktes aus Beispiel 7, 3 g 37 %iger Formal in-Lösung, 0,42 g Eisessig und 0,46 g Natriumcyanoborhydrid wurden analog Beispiel 6 umgesetzt. Das ausgefallene Öl wurde als Oxalat aus Aceton kristallisiert. Man erhielt 1,6 g N,N,N-(2-(4-Cyanobenzyloxy)benzyl)methyl-(2-(4-pyridyl)ethyl)-amino- xalat. Schmp. 161-162 ° C.

### Beispiel 9

15,0 g (54 mmol) des Produktes aus Präparation 6 und 5,3 ml (54 mmol) Piperidin wurden in einem Gemisch aus 50 ml Triethylamin und 200 ml Dimethylformamid bei Raumtemperatur 16 h gerührt. Das Reaktionsgemisch wurde mit viel Wasser verdünnt und mit Ether extrahiert. Die organische Phase wurde getrocknet und mit etherischer Chlorwasserstoff-Lösung versetzt, wobei das Produkt ausfiel. Man erhielt 12,5 g N-(2-(4-Nitrobenzyloxy)-benzyl)-piperidinhydrochlorid. Schmp. 216°C.

### Beispiel 10

3,0 g (15,8 mmol) N-(2-Aminobenzyl)piperidin und 3,1 g (16,7 mmol) 4-Nitrobenzoylchlorid wurden analog Beispiel 1 umgesetzt. Man erhielt 3,4 g 4-Nitro-N-(2-(N-piperidinylmethyl)-phenyl)benzamid. Schmp. 162°C.

### Beispiel 11

2,0 g (8,4 mmol) 2-(4-Cyanbenzyloxy)benzaldehyd, 1,0 g (8,2 mmol) N-Methyl-4-picolylamin, 0,5 g Eisessig und 0,53 g Natriumcyanoborhydrid wurden analog Beispiel 6 in Methanol umgesetzt. Das erhaltene Öl wurde als Fumarat aus iso-Propanol kristallisiert. Man erhielt 2,3 g N,N,N-(2-(4-Cyanphenyloxy)benzyl)-methyl-4-picolylaminfumarat. Schmp. 155-156 °C.

### Beispiel 12

3,0 g (12,5 mmol) des Produktes aus Präparation 4 und 3,0 g (13,5 mmol) 4-Nitrophenylsulfonylchlorid wurden analog Beispiel 3 umgesetzt. Es fiel ein gelber Festkörper an, der aus Toluol umkristallisiert wurde. Man erhielt 2,9 g N-(2(N,N-Methyl-(2-phenylethyl)aminomethyl)phenyl)-4-nitrophenyl-sulfonamid. Schmp. 151-152°C.

## Patentansprüche

1. Phenylbenzylamine der Formel I 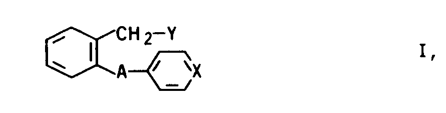
worin bedeuten:
A 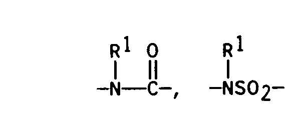
und -O(CH₂)m- (mit Bindung von N bzw. O an den 1,2-Phenylenring) R¹ H und G₁-C₄-Alkyl,
X N und C-R²,
R² N0₂, CN, NHS0₂CH₃, CF₃, OCF₃ und 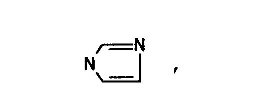
Y NR¹R³ und N (CH₂)ₙ ,
n 4, 5 und 6,
R³ H, G₁-C₄-Alkyl, 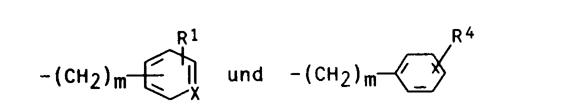
m 1, 2 und 3 und
R⁴ H, OCH₃ und C₁-C₄-Alkyl,
sowie deren physiologisch verträglichen Salze.

2. Arzneimittel zur systemischen Anwendung, das pro Einzeldosis neben üblichen galenischen Hilfsstoffen als Wirkstoff 0,1 bis 50 mg/kg Körpergewicht einer Verbindung nach Anspruch 1 enthält.

3. Arzneimittel zur topischen Anwendung, das neben üblichen galenischen Hilfsstoffen als Wirkstoff 0,001 bis 0,1 Gew.% einer Verbindung nach Anspruch 1 enthält.
